# EUROPEAN PATENT APPLICATION

(11) **EP 1 188 761 A1**
(43) Date of publication of application: **20.03.2002**
(21) Application number: 00308137.9
(22) Date of filing: 18.09.2000
(51) Int. Cl.: C07F 9/38, C07F 9/655, C01B 15/037, C23F 11/167, A61K 31/663, A61K 31/665, A61P 19/00

(54) **Novel geminal-diphosphonic acids and their manufacture**

(71) Applicant: SOLVAY (Société Anonyme), 1050 Bruxelles (BE)
(72) Inventor: Jones, Craig, Cheshire WA5 3DG (GB); Howarth, Suzanne, Cheshire WA1 3NX (GB); Sanderson, William, R, Penketh WA5 2PE (GB)
(74) Representative: Howard, Paul Nicholas

(57) **Abstract**

An α, ω hydroxyl containing compound or its ether formed by reaction of said α, ω hydroxyl groups comprising one or more gem diphosphonic acid groups or salts thereof, characterised in that the α, ω hydroxyl containing compounds are described by the general formula: 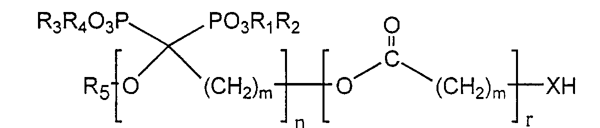
where R₁, R₂, R₃, and R₄ are independently H, Na, or K,
R₅ is hydrogen or an alkylidene radical containing up to 10 carbon atoms and which alkylidene radical comprises at least one hydroxyl substituent
m is an integer equal to 3, 4, 5, or 6
n is ≥ 1
r is ≥ 0
X is either O, 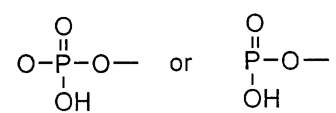 and further where the n + r groups can be distributed randomly throughout the molecule.

## Description

This invention relates to novel geminal-diphosphonic acids and salts thereof. It also relates to methods of manufacture of such compounds and further, to uses of such compounds.

Gem-diphosphonic acids and their salts are well known in the art as being good sequestrants of metal cations such as calcium, magnesium, iron, copper, manganese etc. This has led to their usage in a wide range of applications. For example ethylidene-1-hydroxy-1,1-diphosphonic acid disodium salt, is an efficient stabiliser of hydrogen peroxide and percarboxylic acid solutions towards loss of active oxygen. It is also known to use the above compound as a calcium sequestrant in detergent compositions to reduce the build up of limescale in washing machines. Furthermore, EP 0189662 (The Procter & Gamble Company) discloses the use of cycloalkyl gem-diphosphonic acids and their salts in the treatment of certain bone diseases involving both unwanted deposition of calcium salts in the body and unwanted mobilisation of calcium. WO 96/33199 (Merck & Co. Inc.) discloses 1-hydroxy-1,1-diphosphonic acids generally and 4-amino-1-hydroxybutylidene-1,1-bisphosphonic acid specifically, in the treatment of bone diseases. It also discloses a process for making them. The latter compound is particularly looked on as being effective and several processes are disclosed as being suitable for its production. US 5,019,651 (Merck & Co.) utilises a mixture of phosphorous acid and phosphorous trichloride in the presence of a strong acid to convert the carboxylic acid group of 4-aminobutyric acid to amino-1-hydroxybutylidene-1,1-bisphosphonic acid. US 4,407,761 (Henkel) discloses the conversion of short chain amino-acids using phosphorous acid and a phosphorus chloride or oxychloride. Furthermore EP 0657462 (Albright and Wilson Limited ) exemplifies azacycloheptane-2,2-diphosphonic acid and azacyclononane-2,2-diphosphonic acid in their disclosure of a method of producing gem-diphosphonic acids from lactams.

The large scale use of amino-phosphorus compounds would however not be desirable as both nitrogen containing and phosphorus containing compounds are known to cause problems in the environment due to eutrophication. The combination in one molecule of both elements is therefore highly undesirable.

It is a first object of certain aspects of the present invention to provide a series of nitrogen free gem-diphosphonic acids based on lactones or the corresponding hydroxyacid, and which are based on readily available starting materials. According to further aspects of the invention gem-diphosphonic acids based on polymeric derivatives of the lactones are provided.

It is a second object of further aspects of the present invention to provide a process for the production of such nitrogen free gem-diphosphonic acids.

It is a third object of yet further aspects of the present invention to provide uses for the gem-diphosphonic acids.

According to the present invention, there are provided α, ω hydroxyl containing compounds or their ether formed by reaction of said α, ω hydroxyl groups comprising one or more gem diphosphonic acid groups or salts thereof, characterised in that the α, ω hydroxyl containing compounds are described by the general formula: 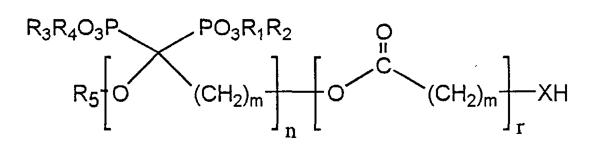 where
R₁, R₂, R₃, and R₄ are independently H, Na, or K,
R₅ is hydrogen or an alkylidene radical containing up to 10 carbon atoms and which alkylidene radical comprises at least one hydroxyl substituent
m is an integer equal to 3, 4, 5, or 6
n is ≥ 1
r is ≥ 0
X is either O, 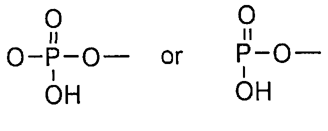
and further where the n + r groups can be distributed randomly throughout the molecule.

Although not wishing to be tied to a particular theory, it is believed that due to amongst others, entropy considerations, sequestration of a metal ion is most efficient when the ligands come from the same molecule, and are especially efficient when the ligating groups are on neighbouring parts of the molecule. Compounds with the above structure where m = 5 are therefore preferred as this allows the molecule to bend easily into a configuration allowing ligation from both one of the gemdiphosphonic acid groups and the nearest either phosphonic acid or carbonyl group on the same molecule. A particularly preferred compound is oxocycloheptane-2,2-diphosphonic acid, either in acid form or as its sodium salt. A further particularly preferred compound is 1,6-dihydroxy-1,1-diphosphonohexane or sodium salts thereof, which compounds can be formed by hydration of the ether group of oxocycloheptane-2,2-diphosphonic acid. In a yet further aspect of the invention preferred compounds are those where at least one of R₁₋₄ are sodium ions.

According to further aspects of the present invention there is provided a process for the production of gem diphosphonic acids or salts thereof comprising reacting the substrate with phosphorous acid or ester thereof and a phosphorus halide at elevated temperature, characterised in that the substrate is an α, ω hydroxyl containing compound or its ether formed by reaction of said α, ω hydroxyl groups and that the α, ω hydroxyl containing compounds are described by the general formula: 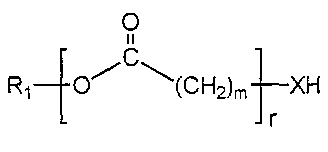
R₁ is hydrogen or an alkylidene radical containing up to 10 carbon atoms and which alkylidene radical comprises at least one hydroxyl substituent
m is an integer equal to 3, 4, 5, or 6
r is ≥ 1
X is either O,

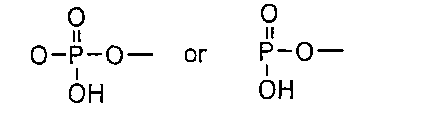

Generally, the substrate is reacted at elevated temperature with a phosphorus(III) acid or alkyl ester thereof and a phosphorus(III)/(V) halide until the product has formed. Water can then be added and if necessary heating applied for a further period of time. The reaction mix is cooled to ambient and any solid filtered off. Distillation is then carried out to remove hydrogen halide formed as a by-product during the reaction. The resulting product is then dissolved in water and the pH optionally adjusted with base. The mix is then poured into a suitable organic solvent in which the product is insoluble, optionally cooled, and the resulting precipitate filtered and washed with further portions of the solvent to yield the desired product. The product can then be characterised by the usual methods.

The phosphorus(III) acid can be added in the form of a low molecular weight ester such as triethylphosphite, it is preferable however to use phosphorus(III) acid. The phosphorus halide is preferably selected from phosphorus(III) chloride, phosphorus(V) chloride, phosphorus(III) bromide, phosphorus(V) bromide, phosphorus(III) iodide, and phosphorus(V) iodide. Phosphorus(III) chloride, phosphorus(V) chloride are particularly preferred. The reaction between the substrate, phosphorus halide and phosphorus acid or ester is carried out at elevated temperature, preferably at reflux until the reaction product has been formed. This is often for a period of up to 8 hours preferably up to about 4 hours. Water can then be added, especially if an ester of phosphorus acid has been used, and the reaction mixture held at elevated temperature, preferably reflux, for a further period of time until any hydrolysis of reaction products has been formed. The base used to adjust the pH can be any of the bases commonly used in the laboratory, but sodium hydroxide is particularly preferred. A concentration of 10%w/w has been found to be suitable. The final pH of the solution upon addition of the base will of course be determined by the degree desired of replacement of H on the phosphonic acid groups by another counterion.

The gem-diphosphonic acids according to the invention are suitable as sequestrants and/or corrosion inhibitor. They can therefore be used in all applications where the sequestrant activity and/or the corrosion inhibiting properties of these compounds are valuable, like stabilisation of peroxygen systems (which, in turn, is valuable in applications as textile bleaching, paper pulp bleaching, and many others), detergent additives, treatment of certain diseases caused by the imbalance of metal cations in the body, cleaning compositions, cosmetic and toiletries, boiler water treatment, cooling water treatment, desalination, oil recovery etc.

Consequently, according to yet further aspects of the invention, there are provided uses for the compounds according to the invention as sequestrant and/or corrosion inhibitor. A first such use is in the stabilisation of peroxygen systems, which in turn is valuable in fields such as textile bleaching, paper pulp bleaching, and many others. Other such uses are in detergent compositions, treatment of certain diseases caused by the imbalance of metal cations in the body, cleaning compositions, cosmetic and toiletries, boiler water treatment, cooling water treatment, desalination and oil recovery.

The peroxygen compounds likely to be stabilised with the compounds according to the invention are preferably inorganic peroxygen compounds, like hydrogen peroxide, and water soluble organic peroxygen compounds, like low molecular weight aliphatic peroxyacids, for example containing up to 6 carbon atoms, like peracetic acid.

Having described the invention in general terms, specific embodiments thereof are described in greater detail by way of example only.

### Example 1: Preparation of oxocycloheptane-2,2-diphosphonic acid, sodium salt

ε-caprolactone (30g, 0.26 moles) and phosphorous acid (47.2g, 0.54 moles) were charged to a flask and phosphorous trichloride (47ml, 0.57 moles) added slowly. The mixture was then refluxed for 4 hours, after which time a thick orange by-product had formed. Demineralised water (200ml) was then added slowly and the suspension refluxed for a further 2 hours. The mixture was then cooled to ambient and, following filtration to remove the orange solid, the hydrochloric acid generated during the reaction distilled off. The resultant thick water white viscous oil (60g) was then split into two and one half of it dissolved in water (100ml). The pH was adjusted to 5 with sodium hydroxide (10%w/w) and the solution decanted into ice cold methanol. A white precipitate formed which was filtered off, washed with 3 x 100ml portions of cold methanol, and dried under vacuum for 18 hours to yield 35g of product.

Phosphorus-31 n.m.r. of the precipitate showed a peak at a shift of δ = -20.2 ppm with respect to H₃PO₄ (85%w/w), with further peaks at -1.4, -3.9, and -7.8 ppm. The peak at -20.2 ppm corresponds to the gem-diphosphonic group.

Carbon-13 n.m.r. of the precipitate showed a triplet at δ = 77.0 with respect to TMS corresponding to a tertiary carbon bearing gem-diphosphonic groups.

Phosphorous-31 n.m.r. of the oil showed a peak at a shift of δ = -20.4 ppm with respect to H₃PO₄ (85%w/w), with further peaks at -0.7, -4.9, and -6.2 ppm.

### Example 2: Preparation of oxocycloheptane-2,2-diphosphonic acid

The method of Example 1 was applied except that there was no pH adjustment after dissolution of the water white oil in water. Oxocycloheptane-2,2-Diphosphonic acid was obtained in 60g yield.

Phosphorus-31 n.m.r. showed a peak at a shift of δ = -20.4 ppm with respect to H₃PO₄ (85%w/w), with further peaks at -0.7, -4.9, and -6.2 ppm. The peak at -20.4 ppm corresponds to the gem-diphosphonic acid group. Carbon-13 n.m.r. showed a triplet at δ = 76.0 with respect to TMS corresponding to a tertiary carbon bearing gem-diphosphonic acid groups.

### Example 3: Gem-phosphonic acid, sodium salt derivative of CAPA 203®

The experimental method given in Example 1 was applied to CAPA 203® (polycaprolactone, number average molecular mass 400). 32g of product was obtained.

Phosphorus-31 n.m.r. showed a peak at a shift of δ = -20.2 ppm with respect to H₃PO₄ (85%w/w), with further peaks at -2.3 and -3.7 ppm. The peak at -20.2 ppm corresponds to the gem-diphosphonic acid group.

Carbon-13 n.m.r. showed a triplet at δ = 74.0 with respect to TMS corresponding to a tertiary carbon bearing gem-diphosphonic acid groups.

### Example 4: Gem-phosphonic acid, sodium salt derivative of CAPA 316®

The experimental method given in Example 1 was applied to CAPA 316® (polycaprolactone, number average molecular mass 1000). 38g of product was obtained.

### Example 5: Gem-phosphonic acid, sodium salt derivative Carboxylate Capped Polycaprolactone

The experimental method given in Example 1 was applied to a polycaprolactone with an end-carboxyl group (number average molecular mass 260). 29g of product was obtained.

Phosphorus-31 n.m.r. showed two peaks at shifts of δ = -19.8 and -20.6 ppm with respect to H₃PO₄ (85%w/w), with further peaks at -3.7 and -6.1 ppm. The peaks at -19.8 and -20.6 ppm correspond to two distinct gem-diphosphonic acid groups.

### Calcium Sequestration Results

**Table 1:**

| Calcium Sequestration Results at 20g Calcium / 100g Sequestrant | |
|---|---|
| Material | % Calcium Sequestered |
| Azacycloheptane-2,2-diphosphonic acid | 76.0 |
| Sodium tripolyphosphate | 93.1 |
| Example 1 | 92.1 |
| Example 2 | 91.0 |
| Example 3 | 94.7 |
| Example 5 | 97.5 |
| BRIQUEST ADPA-60A (Ethylidene-1-hydroxy-1,1-diphosphonic acid sodium salt) | 77.9 |

From these results it can clearly be seen that the compounds according to the present invention are as good as the leading sequestrant, sodium tripolyphosphate, and significantly better than the widely used BRIQUEST. Comparison of Example 2 with azacycloheptane-2,2-diphosphonic acid also shows the surprising and previously undisclosed improvement obtained on basing the sequestrant on a lactone instead of a lactam.

## Claims

1. An α, ω hydroxyl containing compound or its ether formed by reaction of said α, ω hydroxyl groups comprising one or more gem diphosphonic acid groups or salts thereof, **characterised in that** the α, ω hydroxyl containing compounds are described by the general formula: 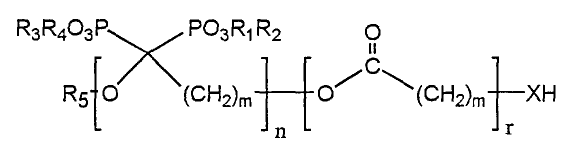 where
R₁, R₂, R₃, and R₄ are independently H, Na, or K,
R₅ is hydrogen or an alkylidene radical containing up to 10 carbon atoms and
which alkylidene radical comprises at least one hydroxyl substituent
m is an integer equal to 3, 4, 5, or 6
n is ≥ 1
r is ≥ 0
X is either O, 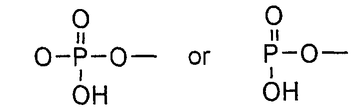
and further where the n + r groups can be distributed randomly throughout the molecule.

2. A compound according to claim 1, **characterised in that** m = 5.

3. A compound according to claims 1 and 2, **characterised in that** r = 0 and n = 1.

4. A compound according to any preceding claim, **characterised in that** X=O.

5. A compound according to any preceding claim, **characterised in that** R₅ = H.

6. A compound according to claim 1, **characterised in that** m = 5, r = 0, n = 1, X = O, and R₅ = H and that the compound is the ether formed by reaction of the α and ω hydroxyl groups.

7. A process for the production of gem diphosphonic acids or salts thereof comprising reacting the substrate with phosphorous acid or ester thereof and a phosphorus halide at elevated temperature, **characterised in that** the substrate is an α, ω hydroxyl containing compound or its ether formed by reaction of said α, ω hydroxyl groups and that the α, ω hydroxyl containing compounds are described by the general formula: 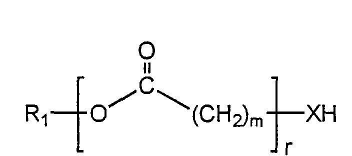
R₁ is hydrogen or an alkylidene radical containing up to 10 carbon atoms and which alkylidene radical comprises at least one hydroxyl substituent
m is an integer equal to 3, 4, 5, or 6
r is ≥ 1
X is either O,

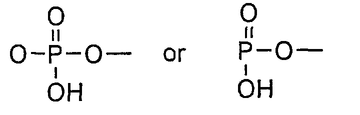

8. Use of the compounds according to claims 1 to 6 as a sequestrant and/or corrosion inhibitor.

9. Use according to claim 8 in the stabilisation of peroxygen systems.

10. Use according to claim 8 in detergent compositions, treatment of certain diseases caused by the imbalance of metal cations in the body, cleaning compositions, cosmetic and toiletries, boiler water treatment, cooling water treatment, desalination and oil recovery.
